# EUROPEAN PATENT SPECIFICATION

(11) **EP 4 088 585 B1**
(45) Date of publication and mention of the grant of the patent: **18.10.2023**
(21) Application number: 21173540.2
(22) Date of filing: 12.05.2021
(51) Int. Cl.: A23J 3/22, A23L 31/00, C12N 1/12, C12N 1/14

(54) **FISH AND SEAFOOD SUBSTITUTE MADE FROM FIBROUS MYCELIUM AND MICROALGAE AND METHODS OF PRODUCTION THEREOF**
FISCH- UND MEERESFRÜCHTEERSATZ AUS FASERIGEM MYZEL UND MIKROALGEN UND VERFAHREN ZU DEREN HERSTELLUNG
SUBSTITUT DE POISSON ET DE FRUITS DE MER FABRIQUÉ À PARTIR DE MYCÉLIUM FIBREUX ET DE MICROALGUES ET SES PROCÉDÉS DE PRODUCTION

(43) Date of publication of application: 16.11.2022
(73) Proprietor: Koralo GmbH, 85598 Baldham (DE)
(72) Inventor: Stedman, Michael Pheroze, Marlborough Wiltshire SN8 2EN (GB)
(74) Representative: Patentanwälte Dr. Keller, Schwertfeger Partnerschaft mbB

(56) References cited:
- WO-A1-2018/202852
- WO-A1-2020/232347
- US-A1- 2012 282 651
- US-A1- 2019 373 934
- CAO WEIXING ET AL: "Simultaneously upgrading biogas and purifying biogas slurry using cocultivation ofChlorella vulgarisand three different fungi under various mixed light wavelength and photoperiods", BIORESOURCE TECHNOLOGY, ELSEVIER, AMSTERDAM, NL, vol. 241, 1 June 2017 (2017-06-01), pages 701-709, XP085129403, ISSN: 0960-8524, DOI: 10.1016/J.BIORTECH.2017.05.194

## Description

### FIELD OF THE INVENTION

The invention is in the field of edible non-animal fish and seafood replacement products and food supplements. The main ingredients of the edible non-animal fish and seafood substitute products of the present invention are derived from fibrous mycelium mass and micro- or macroalgae. The invention also describes the methods of producing these food products and food supplements.

### BACKGROUND

As the largest traded food commodity in the world, seafood provides sustenance to billions of people worldwide. Approximately 3 billion people in the world rely on wild-caught and farmed seafood as a primary source of protein.

Historically, the seafood industry has significantly impacted the environment. The United Nations Food and Agricultural Organization estimates that 85% of marine fish stocks are either fully exploited or overfished [1]. On top, most of the existing fishing practices have a very high negative toll by destroying whole ecosystems with their fishing nets and not being able to fish selectively certain target species. The resulting bycatch is widely used as animal feed in aquaculture and for farmed animals such as chicken, which is a highly ineffective practice for producing protein.

Aquaculture cannot replace wild fishing as it also heavily uses protein from fishes caught in the wild as bycatch or dedicated such as anchovy, which are then converted into feed.

These problems of non-sustainability of fish and seafood harvesting and aquaculture will be aggravated by a human population growing to 9.6 bn by 2050, expecting to leave more than 170 countries with substantial unmet demand.

Fish and seafood is also considered a very healthy alternative to animal protein because it has no cholesterol, is low in fat and a lot of fish have larger amounts of non-saturated essential fatty acids, in particular ALA, EPA and DHA. On top of that many fish and seafood varieties deliver vitamin D [2]. EPA and DHA are not produced by the species representing fish or seafood (fish including seawater species such as salmon, cod, tuna, haddock and others or sweet-water fish such as trout, pangasius, carp or others; shrimp; scallops; crabs; lobster; mussels, clams) themselves. They are taken up through the food chain: large fish eat small fish, small fish eat crustaceans and crustaceans eat microalgae. The microalgae synthesize these essential fatty acids. Crustaceans are part of the group of so called filter feeders to which also clams and mussels belong, which feed directly on microalgae as do some fish species.

While in recent years there have been more and more products launched as meat replacements based on plant protein, there have been little alternative options for seafood replacements. The majority of existing plant-based fish and seafood alternatives are using plant-based proteins such as soy, wheat, chick pea, mung bean or pea and their respective protein isolates. These proteins or protein isolates are then mixed with flavors or flavor-imparting materials, amongst others macroalgae to mask the off-flavor of the original protein isolates and to give a fish or seafood flavor. In some cases vegetable oils are added to improve mouthfeel. After adding binding agents or texturizers to the raw materials, the mix is then heavily processed, e.g. with extrusion processes, to achieve a firm texture.

US2019254328A1 describes an algae-based food product containing an algae-based cocoa butter, an algae-based powder, or flour that contains protein and fiber, and optionally at least one flavoring agent, a sweetener or a fiber powder.

US2020060309A1 describes a foodstuff having a fibrous structure comprising at least one plant constituent of aquatic origin selected from among microalgae or microalgae extracts, cyanobacteria or cyanobacteria extracts and marine fungi or marine fungi extracts of the genus Schizochytrium and Auriantochytrium, or a mixture of at least two of these constituents of aquatic origin, and at least one other plant constituent.

Cao et al. describe cocultivation of the algea *Chlorella vulgaris (C. vulgaris)* and three different fungi *Pleurotus geesteranus (P. geesteranus), Ganoderma lucidum (G. lucidum),* and *Pleurotus ostreatus (P. ostreatus)* in order to purify biogas slurry and biogas simultaneously [3].

### SUMMARY OF THE INVENTION

Against this background, it is the object of the present invention to provide a fish and seafood replacement product or food supplement for human consumption, which is not based on wild harvest or aqua culture.

This object is solved by a composition comprising
a) a fibrous mycelium mass of at least one edible fungal strain in the range of 0.5 wt% to 99.5 % wt%,
b) water in the range of up to 99.5 wt%,
c) edible micro- or macroalgae or parts thereof, wherein the edible micro- or macroalgae or the parts thereof and the fibrous mycelium mass were incubated together in a growth medium for the edible fungal strain,whereinthe composition comprises one or more of the following additional components:
d) a flavoring component selected from herbs, spices or extracts of them, sauces, natural, artificial or nature-identical flavors,
e) a texturizing component selected from starch, gum, algae- derived component or methyl cellulose,
f) a preserving component selected from acids, salts, natural antioxidants or artificial preservatives,
g) a carbohydrate component derived from cereals, tubers or bulbo tubers,
h) a fibre component selected from fruits, algae or vegetables,
i) a coloring component derived from spice or vegetable extracts, algae, vegetable oils, artificial or nature-identical color, pigments,
j) a protein component selected from soy, pea, wheat, rice, lupin, mung bean and chick pea,
k) a plant-derived lipid component selected from rapeseed, linseed, coconut, canola, sunflower, olive, algae and palm.

The present invention solves the challenges of non-sustainable animal-based seafood and plant-based seafood at the same time by modifying an existing raw material introduced as meat replacement namely mycelia of edible fungi.

Although plant-based fish and seafood replacement products containing mycelia of edible fungi, specifically Fusarium venenatum, are being sold, they are nutritionally far inferior to the animal-based products they aim to replace containing no polyunsaturated fatty acids such as DHA or EPA and no vitamin D. Furthermore, they require additional flavoring to mimic the fish and seafood flavor [4; 5].

Micro- and macroalgae or their components such as Omega-3-fatty acids, in particular EPA and DHA, have also been marketed as food supplements or additives. One of the challenges in the production process of micro-algae derived components has been the separation of micro-algae from the growth medium. Next to classical separation techniques like filtration or centrifugation, flocculation with fungi has been successfully deployed in the production of microalgae for biofuels and for lipid extraction [6 - 8].

In the present invention it has been surprisingly found that mycelia of edible fungi are able to digest micro- and macroalgae, absorbing non- and polyunsaturated fats, amongst them omega-3-fatty acids such as ALA and DHA from the algae, and forming vitamin D in high quantities when exposed to light. The nutritional profile of the obtained edible fungi is very similar to animal-based fish and seafood, which makes them a superior alternative to existing plant-based products. Cultivation in a closed system and the direct feeding of algae to mycelia avoids the accumulation of heavy metals like mercury compared to traditional fish and seafood, which is an additional advantage. The isolated mycelium mass has a natural fish and seafood flavor and a texture similar to the one of fish and seafood.

In one embodiment of the present invention microalgae are grown as described in prior art, separated from their growth liquid, sterilized if the microbiological contamination requires, their cell wall structure disrupted through physical forces and/or enzymatically treated and added to a fungal mycelium in a batch, fed-batch or continuous fermentation bioreactor. In the case of macroalgae these are sterilized, crushed and their cell wall structure disrupted through physical forces and/or enzymatically treated before addition to the culture medium of the fungal mycelium. Combining different quantities of different micro- or macroalgae gives a mixture of carbohydrates and proteins optimum for the growth of the fungal mycelia. The mixture of different microalgae also provides different fatty acid profiles aiding to the concentration of omega-3-fatty acids such as EPA and DHA in the fungal mycelium. Optionally other growth factors and process aids known to the skilled person not provided by the micro- and macroalgae to obtain optimum growth conditions are added. After completing their growth the fungal mycelia are separated from the liquid in the bioreactor. The remaining liquid in the fermentation bioreactor or any washing waters are recycled by supplementing them as a growth medium to the micro- or macroalgae. Remaining nutrients help the micro- or macroalgae to grow and make the complete system very eco-friendly.

In one embodiment microalgae and fungal mycelia from edible fungi are grown in one bioreactor together. A symbiotic system is created, where microalgae with the help of light, CO₂, trace elements such as Fe and an N-, P- and optionally a Si-source produce organic matter and oxygen. The fungal mycelia consume the oxygen and feed on the organic matter of the microalgae producing CO₂. The CO₂ stimulates the growth of microalgae. The fungal mycelia are separated by filtration and the microalgae remain suspended in the liquid because of their small size. Like in the first embodiment, optionally other growth factors and process aids known to the skilled person not provided by the microalgae are added to obtain optimum growth conditions for the fungal mycelia. The light required for growth of microalgae can be provided by artificial lights in a metallic vessel or through natural daylight / artificial light in a transparent bioreactor. In a specific embodiment this can be a photobioreactor, such as a tubular or panel system with liquid flow and temperature control system for optimum temperature and gas transfer. The system of this embodiment will be even more eco-friendly than the first embodiment as it uses less energy for maintaining a homogeneous mixture of nutrients and gases in the bioreactor.

In one embodiment microalgae and fungal mycelia from edible fungi are grown in one bioreactor together, where the microalgae are separated temporarily from the main system and their cell walls disrupted by physical forces or enzymatically treated and re-looped into the main system as more accessible feed stock for the fungal mycelia.

In one embodiment microalgae are first grown in a separate system to be added with their growing liquid to the main vessel where microalgae and fungal mycelia from edible fungi are grown together. This embodiment is favorable when microalgae in different concentrations and with different optimum growth conditions are required for the optimum growth of the fungal mycelia or to obtain a specific nutritional or flavor profile.

In one embodiment the components such as proteins, lipids, carbohydrates or pigments are extracted from micro- or macroalgae and added to the culture medium of fungal mycelia.

The isolated fungal mycelium of the edible fungi from all of the embodiments is suitable as an ingredient for plant-based fish and seafood food products or as a nutritional supplement high in vitamin D if exposed to UV-B radiation.

### DESCRIPTION OF THE INVENTION

The term "edible" as used in the context of the present invention relates to a food product or food supplement that is suitable for human consumption.

The term "non-animal" in the context of "non-animal fish and seafood replacement products" relates to a food product or food supplement that comprises mono- and poly-unsaturated fatty acids, such as ALA (alpha-linolenic acid) and DHA (docosahexaenoic acid) and Vitamin D, but that is not based on wild harvest or aquaculture.

The term "fibrous mycelium mass" relates to the mycelia of at least one edible fungal strain.

The mycelia of edible fungi are derived from heterotrophic organisms requiring organic material and oxygen for growth. The mycelium is the vegetative part of a fungus or fungus-like bacterial colony, consisting of a mass of branching. As such it is an extension of the hyphae of fungi that emanates from a fungal spore.

Micro- or macroalgae selected for this invention are photoautotrophic and can also be grown under heterotrophic or mixotrophic conditions.

The described plant-based fish and seafood replacement ingredient consists of the mycelia of edible fungi, which have been grown in a fermentation liquid which contained micro- or macroalgae or components derived from them.

It is characterized by a natural fish or seafood flavor, which is influenced by the amount, species and the process of providing micro- or macroalgae to the growth medium of the fungal mycelium.

It is further characterized - based on a 100 % dry weight - by a protein content between 15 - 60 %, preferably between 20 - 45 % and most preferably between 25 - 35 %, a lipid content between 1 - 15 %, preferably between 2 - 10 %, a content of mono- and polyunsaturated fatty acids of 0.3 - 10 %, preferably between 1 - 6 % and a vitamin D content between 0.1 - 500 mcg / 100g, preferably between 50 - 350 mcg / 100g.

The fish and seafood replacement ingredient is further characterized by a white, yellow, brown or red color influenced by the amount, species and the process of providing micro- or macroalgae to the culture medium of the fungal mycelium.

In a preferred embodiment, the non-animal fish and seafood replacement product or food supplement for human consumption according to the present invention is characterized in that the edible micro- or macroalgae or the parts thereof and the fibrous mycelium mass in the composition were incubated together in the growth medium for the edible fungal strain for at least 60 minutes. Preferably, the incubation time of the composition of the fibrous mycelium mass of the edible fungal strain takes more than 10 hours, preferably between 24 and 216 hours.

Fish or seafood replacement food products can be any food product claiming similarity or substitution of fish including seawater finfish species such as salmon, cod, tuna, haddock and others or sweet-water finfish such as trout, pangasius, carp or others; both salt and sweet water-based shrimp, scallops, crabs, lobster and other shellfish, mussels and clams.

Derivatives can be, but are not limited, to fillets, breaded products, minces or dishes or prepared meals based on these derivatives. It also includes food processing and preservation steps such as pasteurizing, sterilizing, acidifying, chilling, freezing or fermenting.

The invention also relates to the use of a composition comprising
a) a fibrous mycelium mass of at least one edible fungal strain in the range of 0.5 wt% to 99.5 % wt%,
b) water in the range of up to 99.5 wt%,
c) edible micro- or macroalgae or parts thereof, wherein the edible micro- or macroalgae or the parts thereof and the fibrous mycelium mass were incubated together in a growth medium for the edible fungal strain as non-animal fish and seafood replacement product or food supplement for human consumption, wherein the composition comprises one or more of the following additional components:
d) a flavoring component selected from herbs, spices or extracts of them, sauces, natural, artificial or nature-identical flavors,
e) a texturizing component selected from starch, gum, algae- derived component or methyl cellulose,
f) a preserving component selected from acids, salts, natural antioxidants or artificial preservatives,
g) a carbohydrate component derived from cereals, tubers or bulbo tubers,
h) a fibre component selected from fruits, algae or vegetables,
i) a coloring component derived from spice or vegetable extracts, algae, vegetable oils, artificial or nature-identical color, pigments,
j) a protein component selected from soy, pea, wheat, rice, lupin, mung bean and chick pea,
k) a plant-derived lipid component selected from rapeseed, linseed, coconut, canola, sunflower, olive, algae and palm.

The present invention also relates to methods of producing a non-animal fish and seafood replacement product or food supplement for human consumption, comprising the steps of
a. incubating a composition of mycelia of at least one edible fungal strain and edible micro- or macroalgae or parts thereof in a liquid growth medium,
b. culturing the composition under conditions that allow growth of the mycelia to produce a fibrous mycelium mass,
c. separating the fibrous mycelium mass of the edible fungal strain from the edible micro- or macroalgae or parts thereof after completion of the growth of the mycelia to obtain the non-animal fish and seafood replacement product or food supplement for human consumption.

As explained in more detail below, additional ingredients can be added to the culture mixture consisting of the fibrous mycelium mass and the edible micro- or macroalgae or parts thereof. Ingredients include, but are not limited to growth factors, trace elements, CO₂, oxygen or light.

For further processing of the ingredient into a plant-based fish or seafood replacement food product the water content of the ingredient can be reduced, it can be combined with other food-grade raw materials suitable for food production or it can undergo typical food processing steps, but not limited to, such as cooking, frying, freezing, cooling, mixing, coating, forming, fermenting or blanching.

The reduction of water content can be achieved by centrifuging, filtering, pressing and evaporating water by applying heat or reducing atmospheric pressure. For applications of the ingredient as a food supplement the water content is between 0.5 - 15 %, preferably between 2 - 9 %. For applications of the ingredient as a flavor additive the water content is between 3 - 30 %, preferably between 5 - 25 %. For applications of the ingredient as a key ingredient in a fish or seafood to give texture as well the water content is between 25 - 98 %, preferably between 65 - 95 %.

Food-grade materials which are suitable to be combined with this fish and seafood ingredient to produce plant-based fish and seafood food products are plant-based proteins, flavoring, texturizing and coloring agents, carbohydrates, lipids, preservatives and fibres. Plant-based proteins can be, but are not limited to soy, pea, wheat, rice, lupin, mung bean and chick pea. Flavoring agents can be herbs and spices or extracts made of them, algae or extracts of them, sauces, which can be potentially fermented such as soy sauce or any other natural, nature-identical or artificial flavoring substances. Texturizing agents can be, but are not limited to alkyl cellulose such as methyl cellulose or its derivatives, starches derived, but not limited to, from potato, rice, wheat, corn or tapioca; gums such as, but not limited to, locust bean gum, carrageenan or other algae derived texturizers. Coloring agents can be any food-grade coloring of natural origin such as spice or vegetable extract, represented for e.g. by curcuma or carrot, algae derived coloring agents such as astaxanthin, other natural pigments such as ß-carotene or artificial coloring agents. Carbohydrates can be, but are not limited to cereals such as corn, wheat or rice and tubers or bloom-tubers such as potato, tapioca and konjac. Lipids can be any plant-derived lipids such as, but not limited to, rapeseed, linseed, coconut, canola, sunflower, olive, algae and palm oil. Preserving agents can be, but are not limited to, natural acids such as lactic or acetic acid, salt, natural antioxidants such as tocopherol or rosemary extract or artificial preservatives such as potassium sorbate. Fibres from plants that can be used, but are not limited to, such as coconut, pea, navy beans, algae or fruit pomaces such as apple or citrus.

Different methods of obtaining the edible fungal mycelium as a fish and seafood replacement ingredient are described hereunder:
In one embodiment of the present invention microalgae are grown under autotrophic conditions in an open pond / raceway system or in a closed photobioreactor with natural daylight or artificial lighting in a tubular, plate form, plastic bag or variations thereof. The culture medium contains a N-, P- and optionally an additional C- or Si-source and trace elements such as iron known to the skilled person. Instead of mineral sources for these nutrients food-grade waste streams from the food industry such as, but not limited to, molasse or corn-steep liquor can be used. When the desired microalgae density is reached, the microalgae are separated from their growth liquid by existing processes such as filtration or centrifugation, which are not further described here as they do not form part of the invention. After separation the microalgae are sterilized to avoid contamination by other microorganisms of the culture medium of the fungal mycelium and their cell wall structure is disrupted through physical forces (sheer and optionally heat) and/or enzymatically treated to facilitate the digestion of the microalgae before adding to the fungal mycelium culture medium. In the case of macroalgae these are after harvest washed, crushed, sterilized and their cell wall structure disrupted through physical forces and/or enzymatically treated before addition to the culture medium of the fungal mycelium. Combining varying quantities of different micro- or macroalgae gives a mixture of carbohydrates and proteins optimum for the growth of the fungal mycelia and influences the lipid content of the fungal mycelia. The mixture of different microalgae also provides different fatty acid profiles aiding to the concentration of omega-3-fatty acids such as ALA, EPA and DHA in the fungal mycelium. Other organic and inorganic feedstock material, micronutrients and process aids to e.g. regulate the pH, known to the skilled person, which are not provided by the micro- and macroalgae can be added to optimize growth conditions. The fermentation of the fungal mycelium can be performed in a state-of-the-art batch, fed-batch or continuous bioreactor, which can be supplied with compressed air, temperature controlled and able to mix its contents. After completing their growth the fungal mycelia are separated from the liquid in the bioreactor and washed. The remaining liquid in the bioreactor or any washing waters are recycled by supplementing them as a growth medium to the micro- or macroalgae. Remaining nutrients help the micro- or macroalgae to grow and make the complete system very eco-friendly.

In a specific embodiment of this embodiment, the oxygen generated from the fungal mycelia fermentation is fed into a connected photobioreactor in which the microalgae are grown. Optionally, the CO₂ generated from the microalgae is fed into the bioreactor where the fungal mycelia are grown. Preferably, the bioreactor is part of a closed system.

In one embodiment, which differs from the above in that the macro- or microalgae after separation are processed with one or multiple extraction steps as described in literature to isolate their components such as proteins, carbohydrates, lipids or pigments. These components are then added as single components or combined in varying concentrations to the fungal fermentation culture medium after sterilization.

In one embodiment microalgae and fungal mycelia from edible fungi are grown in a closed system together, which can consist of a photobioreactor and / or a conventional closed bioreactor. A symbiotic system is created, where microalgae with the help of light, CO₂, trace elements such as Fe and an N-, P- and optionally a Si- and C-source produce organic matter and oxygen. The fungal mycelia consume the oxygen and feed on the organic matter of the microalgae producing CO₂. The CO₂ in turn stimulates the growth of the microalgae. When a sufficient high concentration of the fungal mycelia is achieved, the fungal mycelium is separated from the microalgae by filtration and discharged for further processing. The microalgae remain suspended in the liquid because of their small size. Other organic feedstock material, micronutrients and process aids, e.g. to regulate the pH, known to the skilled person, which are not provided by the micro- and macroalgae can be added to optimize growth conditions. The light required for growth of microalgae can be provided by artificial lights such as LED lights in a closed bioreactor or through natural daylight /artificial lights in a transparent bioreactor. In a specific embodiment this can be a photobioreactor, such as a tubular, bag or panel system with liquid flow and temperature control for optimum temperature and gas transfer. The system of this embodiment will be even more eco-friendly as the need for ventilation as required for in microalgae production in photobioreactors or the need to supply compressed air in the bioreactor for the fungal mycelium or the microalgae is reduced. The energy use can be further reduced by the diffusion of gases in situ rather than physical mixing throughout the reactor vessel.

In a specific embodiment of this embodiment, the fermentation liquid containing only the microalgae is separated temporarily from the main system and the cell walls of the microalgae are disrupted by physical forces or enzymatically treated and then re-looped into the main system as a more accessible feed stock for the fungal mycelia.

In one embodiment microalgae are first grown autotrophically in a separate system and are then added with their growing liquid to the main system. Here the microalgae and fungal mycelia from edible fungi are grown together in a conventional bioreactor and / or a photobioreactor. This embodiment is favorable when microalgae in different concentrations and with different optimum growth conditions are required. It is also used to target a specific nutritional or flavor profile of the fungal mycelia. After adding the growing liquid containing the microalgae, the fermentation medium of the fungal mycelia in the reactor is adjusted to obtain the optimum growth conditions for the fungal mycelia.

The isolated fungal mycelium of the edible fungi from all of the embodiments is suitable as an ingredient for plant-based fish and seafood or as a nutritional supplement. In case it has been exposed to UV light, it is high in vitamin D.

The present invention also relates to a non-animal fish and seafood replacement product or food supplement for human consumption that is obtainable by the methods described herein, in particular a method comprising the steps of
a. incubating a composition of mycelia of at least one edible fungal strain and edible micro- or macroalgae or parts thereof in a liquid growth medium,
b. culturing the composition under conditions that allow growth of the mycelia to produce a fibrous mycelium mass,
c. separating the fibrous mycelium mass of the edible fungal strain from the edible micro- or macroalgae or parts thereof after completion of the growth of the mycelia to obtain the non-animal fish and seafood replacement product or food supplement for human consumption.

Macroalgae as described in this invention can be all non-toxin producing species, preferably those used for food consumption. If macroalgae are selected from the division Rhodophyta, the classes of Bangiophyceae and of Florideophyceae are preferred. Within the class of Bangiophyceae, the order Bangiales and within that order the family Bangiaceae and within that family the genus Pyropia are preferred and most preferred are the species Yezoensis and Haitianensis.

If macroalgae are selected from the class of Florideophyceae then within that class the order Palmariales and Gigartinales are preferred. If the order Palmariales is selected, the family Palmariaceae and within that family the genus Palmaria are preferred and most preferred the species Palmata and Mollis. If the order Gigartinales is selected, the family Gigartinaceae and Solieriaceae are preferred. Within the family Gigartinaceae the genus Chondrus are preferred and most preferred the species Crispus. Within the family Solieriaceae the genus Kapaphycus and Eucheuma are preferred. If the genus Kapaphycus is selected, Alvarezii is the most preferred species. If the genus Eucheuma is selected, Cottonii is the most preferred species.

If macroalgae are selected from the division Chlorophyta, the class of Ulvophyceae and within that class the order Ulvales and within that order the family Ulvaceae and within that family the genus Ulva are preferred and most preferred are the species Lactuca.

If macroalgae are selected from the division Ochrophyta, the class of Phaeophyceae and within the class the order Laminariales and within that order the family Laminariaceae and within that family the genus Sacharina and Laminaria are preferred. If the genus Sacharina is selected, the most preferred are the species Japonica and Latissima. If the genus Laminaria is selected, the most preferred are the species Digitata.

As microalgae all non-toxin producing species are suitable and in general microalgae consumed as food or used for food ingredients are preferred.

If the microalgae are selected from the division Chlorophyta the class of Trebouxiophyceae, Chlorodentrophyceae and Chlorophyceae are preferred. If the class of Trebouxiophyceae is selected, the order Chlorellales is preferred. If the order Chlorellales is selected, the family Chlorellaceae is preferred. If the family Chlorellaceae is selected, the genus Chlorella is preferred and most preferred is the species Vulgaris. If the class of Chlorophyceae is selected, the order Chlamydomonadales and Sphaeropleales are preferred. If the order Chlamydomonadales is selected, the families of Haematococaceae, Dunaliellaceae and Chlamydomonadaceae are preferred. If the family Chlamydomonadaceae is selected, the genus Chlamydomonas is preferred and most preferred is the species Reinhardtii. If the family of Haematococaceae is selected, the genus Haematococus is preferred and most preferred is the species Pluvialis. If the family of Dunaliellaceae is selected, the genus Dunaliella is preferred and most preferred is the species Salina.

If the order Sphaeropleales is selected, the family of Scenedesmaceae is preferred. If the family Scenedesmaceae is selected, the genus Scenedesmus is preferred and most preferred are the species Dimorphus and Acuminatus.

If the class of Chlorodentrophyceae is selected, the order Chlorodendrales is preferred. If the order Chlorodendrales is selected, the family Chlorodendraceae is preferred. If the family Chlorodendraceae is selected, the genus Tetraselmis is preferred and most preferred are the species Suecica and Striatis.

If the microalgae are selected from the division cyanobacteria, the class of Cyanophyceae is preferred. Within the class of Cyanophyceae, the order Oscillatoriales and Synechococcales are preferred. If the order Oscillatoriales is selected, the family Microcoleaceae is preferred. If the family Microcoleaceae is selected, the genus Arthrospira is preferred and most preferred is the species Platensis. If the order Synechococcales is selected, the family Synechococcaceae is preferred. If the family Synechococcaceae is selected, the genus Synechococcus is preferred.

If the microalgae are selected from the division Haptophyta, the class of Prymnesiophyceae and Pavlovophyceaea are preferred. If the class of Prymnesiophyceae is selected, the order Isochrysidales is preferred. If the order Isochrysidales is selected, the family Isochrysidaceae is preferred. If the family Isochrysidaceae is selected, the genus Isochrysidus is preferred and most preferred is the species Galbana. If the class of Pavlovophyceaea are selected, the order Pavlovales is preferred. If the order Pavlovales is selected, the family Pavlovaceae is preferred.

If the microalgae are selected from the division Ochrophyta the class of Bacillariophyceae, Coscinodiscophyceae, Labyrinthulomycetes, Xantophyceae and Eustigmatophyceae are preferred. If the class of Bacillariophyceae is selected, the order Bacillariales is preferred. If the order Bacillariales is selected, the family Bacillariaceae and Phaeodactylaceae are preferred. If the family Bacillariaceae is selected, the genus Nitzschia is preferred and most preferred is the species Dissipata. If the family Phaeodactylaceae is selected, the genus Phaedactylum is preferred and most preferred is the species Tricornutum.

If the class of Coscinodiscophyceae is selected, the order Thalassiosirales and Biddulphiineae are preferred. If the order Thalassiosirales is selected, the family Skeletonemataceae and Thalassiosiaceae are preferred. If the family Skeletonemataceae is selected, the genus Skeletonema is preferred. If the family Thalassiosiaceae is selected, the genus Pseudonana is preferred.

If the order Biddulphiineae is selected, the family Eupodiscaceae is preferred. If the family Eupodiscaceae is selected, the genus Odontella is preferred and most preferred is the species Aurita.

If the class of Labyrinthulomycetes is selected, the order Labyrinthulales is preferred. If the order Labyrinthulales is selected, the family Thraustochytriaceae is preferred. If the family Thraustochytriaceae is selected, the genus Schizochytrium is preferred.

If the class of Xantophyceae is selected, the order Mischocodcales is preferred. If the order Mischocodcales is selected, the family Pleurochloridaceae is preferred. If the family Pleurochloridaceae is selected, the genus Monodus is preferred and most preferred is the species Subterranea.

If the class of Eustigmatophyceae is selected, the order Eustigmatales is preferred. If the order Eustigmatales is selected, the family Monodopsidaceae is preferred. If the family Monodopsidaceae is selected, the genus Nannochloropsis is preferred and most preferred are the species Gaditana, Oceanica and Oculata.

If the microalgae are selected from the division Rhodophyta, the class of Porphyridiophyceae is preferred. If the class of Porphyridiophyceae is selected, the order Porphyridiales is preferred. If the order Porphyridiales is selected,the family Porphyridiaceae is preferred. If the family Porphyridiaceae is selected, the genus Porphyridium is preferred and most preferred is the species Cruentum.

Edible fungi as described in this invention can be fungal strains selected from the division Basidiomycota or Ascomycota.

If the division of Basidiomycota is selected, the preferred subdivision is Agaromycotina. If the subdivision Agaromycotina is selected, the preferred class is Agaricomycetes.
If the class Agaricomycetes is selected, the order Agaricales, Auriculariales, Boletales, Cantharellales, Polyporales and Russulales are preferred
If the order Boletales is selected, the families Boletaceae and Sclerodermataceae are preferred
If the order Cantharellales is selected, the families Cantharellaceae and Hydnaceae are preferred.

If the order Agaricales is selected, the families Agaricaceae, Fistulinaceae, Lyophyllaceae, Marasmiaceae, Omphalotaceae, Physalacriaceae, Pleurotaceae, Schizophyllaceae, Strophariaceae, and Tricholomataceae are preferred.

If the order Polyporales is selected, the families Ganodermataceae, Meripilaceae, Polyporaceae, and Sparassidaceae are preferred.

If the order Russulales is selected, the families Bondarzewiaceae and Hericiaceae are preferred.

If the order Auriculariales is selected, the family Auriculariaceae is preferred.

If the family Pleurotaceae is selected, the genus Pleurotus is preferred and most preferred is the species Sapidus.

If the division Ascomycota is selected, the subdivision Pezizomycotina is preferred.

If the subdivision Pezizomycotina is selected, the classes Pezizomycetes and Sordariomycetes are preferred.

If the class Pezizomycetes is selected, the order Pezizales is preferred. If the order Pezizales is selected, the families Morchellaceae and Tuberaceae are preferred.

If the class Sordariomycetes is selected, the order Hypocreales and Sordariales are preferred. If the order Hypocreales is selected, the families Cordycipitaceae and Nectriaceae are preferred. If the order Sordariales is selected, the family Sordariaceae is preferred.

The present invention will now be further illustrated by the following examples.

### EXAMPLES

### Example 1 - Cultivation of microalgae:

Chlorella vulgaris and Spirulina (Arthrospira platensis) strains were purchased from Algae Research (Carlsbad, California - USA). Haematococcus pluvialis strain was purchased from Carolina Biological Supply Company (South Carolina - USA).

Microalgae were cultivated in a modified BG-11 medium under sterile conditions. The chemical composition of the modified BG-11 medium was as follows: NaCl (5 g L-1), NaNO3 (1.5 g L-1), K2HPO4 (0.04 g L-1), MgSO4·7H2O (0.075 g L-1), CaCl2·2H2O (0.036 g L-1), citric acid (0.006 g L-1), ferric ammonium citrate (0.006 g L-1), EDTA (ethylenediaminetetraacetic acid) (0.001 g L-1), Na2CO3 (0.02 g L-1), and trace metal mix (1.0 mL). The trace metal mix consisted of H3BO3 (2.86 g L-1), MnCl2·4H2O (1.81 g L-1), ZnSO4·7H2O (0.222 g L-1), NaMoO4·2H2O (0.39 g L-1), CuSO4·5H2O (0.079 g L-1), and CoCl2·6H2O (0.05 g L-1). To start an algal culture, 3 L of BG-11 medium was inoculated with 150 mL of the respective strain from inoculum stock. The culture was kept in a 4 L Erlenmeyer flask used as a photo-bioreactor at 24°C, and LED lamps (Luxceo P6 RGB LED Light 2500K-6500K) were arranged around the photobioreactor to supply illumination with a 12 h photoperiod. Magnetic agitation was provided for mixing.

### Example 2 - Preculture of fungal mycelium

From an agar agar plate fully overgrown with Pleurotus sapidus a 1cm x 1 cm piece is stamped out and transferred to a 250 mL Erlenmeyer flask. It is dispersed with an Ultra-Turrax dispersing unit (T 18 Basic, IKA-Werke, Staufen, Germany) for 20 s at 8500 rpm after 100 mL of a cultivation medium autoclaved at 121 °C for 20 min. was added under sterile conditions. The cultivation medium had the following composition: D - (+) - glucose H2O 30.0 g L-1; 1-asparagine H2O 4.5 g L-1; KH2PO4 1.5 g L-1; MgSO4 7 H2O 0.5 g L-1; Yeast extract 3.0 g L-1; Trace element solution 1.0 mL L-1/ L (FeCl3 6H2O 80 mg L-1), ZnSO4 7H2O 90 mg L-1, MnSO4 H2O 30 mg L-1, CuSO4 5H2O 5 mg L-1; EDTA (ethylenediaminetetraacetic acid) 400 mg L-1 everything is sterile filtered) adjust to pH 6.0 with 2 M NaOH / 2 M H2SO4 solution.

Cultivation was carried out in the absence of light for 6 days in the incubation flask at 24 ° C. with an agitation of 120 rpm).

### Example 3 - Main Culture of fungal mycelium with standard cultivation medium and D-(+)-glucose

To a 500 mL narrow necked Erlenmeyer flask 200 mL of the following culture medium was added (L-aspartate monosodium-hydrate 6.2 g L-1, NH4NO3 2.4 g L-1, KNO3 1.5 g L-1, MgSO4 7H2O 0.5 g L-1, D-(+)-Glucose H2O 15 g L-1, FeCl3 6H2O 80 mg L-1, ZnSO4 7H2O 90 mg L-1, MnSO4 H2O 30 mg L-1, CuSO4 5H2O 5 mg L-1, EDTA 400 mg L-1. The pH was adjusted to pH 6.0 with 2 M NaOH / H2SO4 solution, the flask was closed with cellulose stoppers and autoclaved (121 ° C., 20 min). To the solution 20 mL of the homogenized preculture medium of example 2 was added. The fermentation was stopped after 9 days.

A 7.5 L fermenter (Solaris Genesis SIP Benchtop Bioreactor) was used for larger scale fermentations equipped with a propeller stirrer and an aeration tube.

The fermenter was filled with 5 L of medium, the pH was adjusted to 6.0 with 2 M NaOH / H2SO4 solution and autoclaved. Inoculation was carried out with 500 mL of homogenized preculture from example 2. The fermenter settings were as follows: stirrer speed 150 rpm, temperature: 24 ° C., aeration rate: 0.3 acm H-1. Fermentation was stopped after 7 days.

The cultures were transferred to 250 mL centrifuge beakers and centrifuged for 10 min at 3.2 g. The mycelium was then washed three times with bi-distilled water (deionized water) until the supernatant was colorless. For larger amounts of mycelium, this was alternatively separated from the supernatant via a pass cloth and also washed with deionized water. The resulting fermentation products were freeze-dried at -85 °C (Telstar LyoAlfa 10 - 85 Freeze Dryer) for chemical analysis.

For sensory analysis the washed fermentation product was tasted by a sensory panel uncooked and after frying in a pan with the following observations:
a. Uncooked: color - white, pale yellow; aroma - slight fruity note; taste - neutral, slight savory,
b. Cooked: color - brown from frying; aroma - fried, savory; taste - savory, neutral.

A sample was freeze-dried to a dry matter content of 94.86 %. Standard analytical methods were used to determine the composition of its content and of all other samples.

Protein content was determined according to §64 LFGB L 17.00-15 : 2013-08 (mod.). Fat content was determined according to §64 LFGB L 17.00-4 : 1982-05 (mod.). The fatty acid composition was determined according to DGF C-VI 11a : 2016 (mod.) and DGF C-VI 10a : 2016 (mod.) and calculated as methyl esters. The content of saturated, mono- and polyunsaturated fatty acids was calculated as well.

Ash content was determined according to §64 LFGB L 17.00-3 : 2002-12 (mod.) and water content according to §64 LFGB L 17.00-1 : 2002-12 (mod.).

Fiber content was determined according to §64 LFGB L 00.00-18 : 1997-01.

Sugar content was determined via HPLC according to DIN 10758 : 1997-05 (mod.) and the carbohydrate content calculated via differential method.

The calorific value was calculated according to VO (EU) Nr. 1169/2011.

Vitamin D2 was determined according to DIN EN 12821 : 2009-08 (mod.).

The full composition is shown in table 1.

**Table 1**

| | | |
|---|---|---|
| **Protein** | 25.31 | g/100g dry weight product |
| **Fat** | 2.34 | g/100g dry weight product |
| **Ash** | 9.02 | g/100g dry weight product |

| Carbohydrates | | |
|---|---|---|
| *Total Fibers* | 60.65 | g/100g dry weight product |
| *Soluble* | 3.43 | g/100g dry weight product |
| Insoluble | 57.22 | g/100g dry weight product |
| **Sugar** | 2.68 | g/100g dry weight product |
| Calorific value | 240.35 | kcal/100g dry weight product |
| | 1051.02 | KJ/100 g dry weight product |
| **Vitamin D2** | nd | mcg/100 g dry weight product |
| **Fatty acids** | 2.343 | g/100g dry weight product |
| **Saturated fatty acids** | 1.43 | g/100g dry weight product |
| **Mono-unsaturated fatty acids** | 0.791 | g/100g dry weight product |
| **poly-unsaturated fatty acids** | 0.122 | g/100g dry weight product |
| **Lauric acid C 12:0** | 0.09 | g/100g dry weight product |
| **Myristic acid C 14:0** | 0.1 | g/100g dry weight product |
| **Palmitic acid C 16:0** | 0.71 | g/100g dry weight product |
| **Palmitoleic acid C 16:1 cis (9)** | 0.055 | g/100g dry weight product |
| **Stearic acid C 18:0** | 0.53 | g/100g dry weight product |
| **Octadecenic acid C 18:1 - cis (9)** | 0.054 | g/100g dry weight product |
| **Octadecenic acid C 18:1 - trans (9)** | 0.052 | g/100g dry weight product |
| **Oleic acid C 18:1w9c (9)** | 0.63 | g/100g dry weight product |
| **Linoleic acid C 18:2 (9,12) - cis, cis** | 0.11 | g/100g dry weight product |
| **Octadecadienic acid C 18:2w6-trans** | 0.012 | g/100g dry weight product |
| ***Alpha linolenic acid (ALA) C 18:3w3c (9,12,15)*** | nd | g/100g dry weight product |
| ***Docosahexaenoic acid (DHA) C22:6 (4,7,10,13,16,19)*** | nd | g/100g dry weight product |

### Example 4 - Main Culture of fungal mycelium with ruptured Chlorella vulgaris

The fermentation broth with Chlorella vulgaris from example 1 were transferred to 250 mL centrifuge beakers and centrifuged for 10 min at 3.2 g. The decanted microalgae were transferred into an Erlenmeyer flask and autoclaved at 121 °C for 20 min.. After re-suspension in 50 mL sterilized water the cell walls were ruptured by an Ultra Turrax with 24,000 rpm for 10 min. This suspension was split in two 500 mL narrow necked Erlenmeyer flasks each having 200 mL of the following culture medium (L-aspartate monosodium-hydrate 6.2 g L-1, NH4NO3 2.4 g L-1, KNO3 1.5 g L-1, MgSO4 0.5 g L-1, FeCl3 80 mg L-1, ZnSO4 90 mg L-1, MnSO4 30 mg L-1, CuSO4 5 mg L-1, EDTA 400 mg L-1. The pH was adjusted to pH 6.0 with 2 M NaOH / H2SO4 solution, the flasks were closed with cellulose stoppers and autoclaved (121 ° C., 20 min). To the solution 20 mL of the homogenized preculture medium of example 2 were added.

The fermentation was stopped after 9 days and the fermentation product separated as in example 3.

For sensory analysis the washed fermentation product was tasted by a sensory panel uncooked and after frying in a pan with the following observations:
c. Uncooked: color - white, pale yellow; aroma - slight vegetal note; taste - neutral, slight algae,
d. Cooked: color - brown from frying; aroma - fried, fresh seafood; taste - savory, like very fresh fish, slightly fatty.

A sample was freeze-dried to a dry matter content of 95.78 %. Standard analytical methods were used to determine the composition of its content as described in example 2.

The full composition is shown in table 2.

**Table 2**

| | | |
|---|---|---|
| **Protein** | 23.30 | q/100q dry weight product |
| **Fat** | 5.09 | g/100g dry weight product |
| **Ash** | 8.26 | g/100g dry weight product |

| Carbohydrates | | |
|---|---|---|
| *Total Fibers* | 60.75 | g/100g dry weight product |
| *Soluble* | 2.97 | g/100g dry weight product |
| Insoluble | 57.78 | g/100g dry weight product |
| **Sugar** | 2.48 | g/100g dry weight product |
| Calorific value | 238.05 | kcal/100g dry weight product |
| | 1040.93 | KJ/100 g dry weight product |
| **Vitamin D2** | nd | mcg/100 g dry weight product |
| **Fatty acids** | 5.09 | g/100g dry weight product |
| **Saturated fatty acids** | 2.00 | g/100g dry weight product |
| **Mono-unsaturated fatty acids** | 1.092 | g/100g dry weight product |
| **poly-unsaturated fatty acids** | 1.998 | g/100g dry weight product |
| **Lauric acid C 12:0** | 0.09 | g/100g dry weight product |
| **Myristic acid C 14:0** | 0.1 | g/100g dry weight product |
| **Palmitic acid C 16:0** | 1.28 | g/100g dry weight product |
| **Palmitoleic acid C 16:1 cis (9)** | 0.29 | g/100g dry weight product |
| **Stearic acid C 18:0** | 0.52 | g/100g dry weight product |
| **Octadecenic acid C 18:1 - cis (9)** | 0.12 | g/100g dry weight product |
| **Octadecenic acid C 18:1 - trans (9)** | 0.052 | g/100g dry weight product |
| **Oleic acid C 18:1w9c (9)** | 0.63 | g/100g dry weight product |
| **Linoleic acid C 18:2 (9,12) - cis, cis** | 0.95 | g/100g dry weight product |
| **Octadecadienic acid C 18:2w6-trans** | 0.012 | g/100g dry weight product |
| ***Alpha linolenic acid (ALA) C 18:3w3c (9,12,15)*** | 0.99 | g/100g dry weight product |
| ***Docosahexaenoic acid (DHA) C22:6 (4,7,10,13,16,19)*** | 0.053 | g/100g dry weight product |

### Example 5 - Main Culture of fungal mycelium with ruptured Spirulina

The fermentation broth with Spirulina from example 1 was transferred to 250 ml centrifuge beakers and centrifuged for 10 min at 3.2 g. The decanted microalgae were transferred into an Erlenmeyer flask and autoclaved at 121 °C for 20 min.. After re-suspension in 50 mL sterilized water the cell walls were ruptured by an Ultra Turrax (T 18 Basic, IKA-Werke, Staufen, Germany) with 24,000 rpm for 10 min. This suspension was split in two 500 mL narrow necked Erlenmeyer flasks each having 200 mL of the following culture medium (L-aspartate monosodium-hydrate 6.2 g L-1, NH4NO3 2.4 g L-1, KNO3 1.5 g L-1, MgSO4 0.5 g L-1, FeCl3 80 mg L-1, ZnSO4 90 mg L-1, MnSO4 30 mg L-1, CuSO4 5 mg L-1, EDTA 400 mg L-1. The pH was adjusted to pH 6.0 with 2 M NaOH / H2SO4 solution, the flasks were closed with cellulose stoppers and autoclaved (121 ° C., 20 min). To the solution 20 mL of the homogenized preculture medium of example 2 were added.

The fermentation was stopped after 9 days and the fermentation product separated as in example 3.

For sensory analysis the washed fermentation product was tasted by a sensory panel uncooked and after frying in a pan with the following observations:
a. Uncooked: color - white, pale yellow; aroma - slight vegetal note; taste - neutral, slight algae,
b. Cooked: color - brown from frying; aroma - fried, fresh seafood; taste - savory, like very fresh fish, slightly fatty.

### Example 6 - Main Culture of fungal mycelium with ruptured Haematococcus pluvialis

The fermentation broth with Haematococcus pluvialis from example 1 was transferred to 250 mL centrifuge beakers and centrifuged for 10 min at 3.2 g. The decanted microalgae were transferred into an Erlenmeyer flask and autoclaved at 121 °C for 20 min.. After re-suspension in 50 mL sterilized water the cell walls were ruptured by an Ultra Turrax (T 18 Basic, IKA-Werke, Staufen, Germany) with 24,000 rpm for 10 min. This suspension was split in two 500 mL narrow necked Erlenmeyer flasks each having 200 mL of the following culture medium (L-aspartate monosodium-hydrate 6.2 g L-1, NH4NO3 2.4 g L-1, KNO3 1.5 g L-1, MgSO4 0.5 g L-1, FeCl3 80 mg L-1, ZnSO4 90 mg L-1, MnSO4 30 mg L-1,
CuSO4 5 mg L-1, EDTA 400 mg L-1/ L ). The pH was adjusted to pH 6.0 with 2 M NaOH / H2SO4 solution, the flasks were closed with cellulose stoppers and autoclaved (121 ° C., 20 min). To the solution 20 mL of the homogenized preculture medium of example 2 were added.

The fermentation was stopped after 9 days and the fermentation product separated as in example 3.

Surprisingly, it was found to have a slight pink-white color, which became more intense after removal of water.

For sensory analysis the washed fermentation product was tasted by a sensory panel uncooked and after frying in a pan with the following observations:
c. Uncooked: color - pink-white; aroma - slight vegetal note; taste - neutral, slight algae,
d. Cooked: color - outside brown from frying, inside grey-pink; aroma - fried, fresh seafood; taste - savory, like very fresh fish, slightly fatty.

### Example 7 - Main Culture of fungal mycelium with non-ruptured Chlorella vulgaris in Photobioreactor - mixotrophic conditions

A 4 L Erlenmeyer flask used as a photo-bioreactor at room temperature (22°C), and LED lamps (Luxceo P6 RGB LED Light 2500K-6500K) were arranged around the photobioreactor to supply illumination with a 12 h photoperiod and it was additionally exposed for 2h / day to sunlight to provide enough UV-B rays. Magnetic agitation was provided for mixing and a stopper with an air inlet for oxygen supply. After 15 days of growth of an autotrophic batch of 3 L Chlorella vulgaris the culture medium was supplemented with D-(+)-glucose (5 g L-1), yeast extract (5 g L-1), KH2PO4 (1.0 g L-1), and a sterile trace element solution of 1 mL L-1 of FeCl3 6 H2O 80 mg L-1), ZnSO4 7 H2O 90 mg L-1, MnSO4 H2O 30 mg L-1, CuSO4 5H2O 5 mg L-1; EDTA 400 mg L-1. The pH was adjusted to 6.0 by the addition of 2 M NaOH / 2 M H2SO4 solution and 150 mL of preculture from example 2 was added.

The flask was aerated with a constant air flow of 0.2 acm H-1 and stirred at maximum speed and kept at 24 °C. LED lamps supplied illumination with a 12 h photoperiod. After 7 days fungal mycelia were separated from the supernatant by passing through a pass cloth. Then it was washed three times with bi-distilled water (deionized water) until the supernatant was colorless and no microalgae visible. The fermentation product had a white-yellow color similar to example 3.

For sensory analysis the washed fermentation product was tasted by a sensory panel uncooked and after frying in a pan with the following observations:
e. Uncooked: color - white, pale yellow; aroma - slight vegetal note; taste - neutral, slight algae,
f. Cooked: color - brown from frying; aroma - fried, fresh seafood; taste - savory, like very fresh fish, slightly fatty, all with similar intensity than example 3.

A sample was freeze-dried to a dry matter content of 93.59 %. Standard analytical methods were used to determine the composition of its content as described in example 2.

The full composition is shown in table 3.

**Table 3**

| | | |
|---|---|---|
| **Protein** | 22.88 | g/100g dry weight product |
| **Fat** | 6.61 | g/100g dry weight product |
| **Ash** | 8.45 | g/100g dry weight product |

| Carbohydrates | | |
|---|---|---|
| *Total Fibers* | 60.32 | g/100g dry weight product |
| *Soluble* | 3.05 | g/100g dry weight product |
| Insoluble | 57.27 | g/100g dry weight product |
| **Sugar** | 1.74 | g/100g dry weight product |
| Calorific value | 243.62 | kcal/100g dry weight product |
| | 1065.29 | KJ/100 g dry weight product |
| **Vitamin D2** | 262 | mcg/100 g dry weight product |
| **Fatty acids** | 6.614 | g/100g dry weight product |
| **Saturated fatty acids** | 2.29 | g/100g dry weight product |
| **Mono-unsaturated fatty acids** | 1.412 | g/100g dry weight product |
| **poly-unsaturated fatty acids** | 2.912 | g/100g dry weight product |
| **Lauric acid C 12:0** | 0.08 | g/100g dry weight product |
| **Myristic acid C 14:0** | 0.1 | g/100g dry weight product |
| **Palmitic acid C 16:0** | 1.59 | g/100g dry weight product |
| **Palmitoleic acid C 16:1 cis (9)** | 0.38 | g/100g dry weight product |
| **Stearic acid C 18:0** | 0.52 | g/100g dry weight product |
| **Octadecenic acid C 18:1 - cis (9)** | 0.11 | g/100g dry weight product |
| **Octadecenic acid C 18:1 - trans (9)** | 0.052 | g/100g dry weight product |
| **Oleic acid C 18:1w9c (9)** | 0.87 | g/100g dry weight product |
| **Linoleic acid C 18:2 (9,12) - cis, cis** | 1.32 | g/100g dry weight product |
| **Octadecadienic acid C 18:2w6-trans** | 0.01 | g/100g dry weight product |
| ***Alpha linolenic acid (ALA) C 18:3w3c (9,12,15)*** | 1.52 | g/100g dry weight product |
| ***Docosahexaenoic acid (DHA) C22:6 (4,7,10,13,16,19)*** | 0.062 | g/100g dry weight product |

### Non-patent literature:

[1] https://www.worldwildlife.org/industries/sustainable-seafood#:~:text=Approximately%203%20billion%20people%20in,to%20billions%20of%20people %20worldwide.
[2] https://www.eatingwell.com/article/7821288/is-seafood-healthy/
[3] Cao W, Wang X, Sun S, Hu C, Zhao Y. Simultaneously upgrading biogas and purifying biogas slurry using cocultivation of Chlorella vulgaris and three different fungi under various mixed light wavelength and photoperiods. Bioresour Technol. 2017 Oct;241:701-709. doi: 10.1016/j.biortech.2017.05.194. Epub 2017 Jun 1. PMID: 28618378.
[4] https://www.quorn.co.uk/products/quorn-vegan-fishless-scampi
[5] https://www.quorn.co.uk/products/quorn-veqan-breaded-fishless-fillets
[6] https://patents.google.com/patent/WO2012109375A2/en
[7] https://patents.qoogle.com/patent/WO2019122030A1/en
[8] https://biotechnologyforbiofuels.biomedcentral.com/track/pdf/10.1186/s13068-015-0210-6.pdf

## Claims

1. A non-animal fish and seafood replacement product or food supplement for humans, comprising a composition of
a) a fibrous mycelium mass of at least one edible fungal strain in the range of 0.5 wt% to 99.5 % wt%
b) water in the range of up to 99.5 wt%
c) edible micro- or macroalgae or parts thereof, wherein the edible micro- or macroalgae or the parts thereof and the fibrous mycelium mass were incubated together in a growth medium for the edible fungal strain, **characterized in that** the composition comprises one or more of the following additional components:
d) a flavoring component selected from herbs, spices or extracts of them, sauces, natural, artificial or nature-identical flavors,
e) a texturizing component selected from starch, gum, algae- derived component or methyl cellulose,
f) a preserving component selected from acids, salts, natural antioxidants or artificial preservatives,
g) a carbohydrate component derived from cereals, tubers or bulbo tubers,
h) a fibre component selected from fruits, algae or vegetables,
i) a coloring component derived from spice or vegetable extracts, algae, vegetable oils, artificial or nature-identical color, pigments,
j) a protein component selected from soy, pea, wheat, rice, lupin, mung bean and chick pea,
k) a plant-derived lipid component selected from rapeseed, linseed, coconut, canola, sunflower, olive, algae and palm.

2. The non-animal fish and seafood replacement product or food supplement according to claim 1, wherein the fibrous mycelium mass has a protein content between 15 - 60 wt%, preferably between 20 - 45 wt% and most preferably between 25 - 35 wt%, a lipid content between 1 - 15 wt%, preferably between 2-10 wt%, a content of mono- and polyunsaturated fatty acids of 0.3 - 10 wt%, preferably between 1 - 6 wt% and a vitamin D content between 0.1 - 500 mcg / 100 g, preferably between 50 - 350 mcg / 100 g.

3. The non-animal fish and seafood replacement product or food supplement according to claim 1 or claim 2, wherein the edible micro- or macroalgae or the parts thereof and the fibrous mycelium mass in the composition were incubated together in the growth medium for the edible fungal strain for at least 60 minutes.

4. A method of producing a non-animal fish and seafood replacement product or food supplement for humans, comprising the steps of:
a. incubating a composition of mycelia of at least one edible fungal strain and edible micro- or macroalgae or parts thereof in a liquid growth medium,
b. culturing the composition under condition that allow growth of the mycelia to produce a fibrous mycelium mass,
c. separating the fibrous mycelium mass of the edible fungal strain from the edible micro- or macroalgae or parts thereof after completion of the growth of the mycelia to obtain the non-animal fish and seafood replacement product or food supplement for humans.

5. The method of producing a non-animal fish and seafood replacement product or food supplement for humans according to claim 4, wherein additional growth factors, trace elements, CO₂, oxygen or light is/are added to the liquid growth medium.

6. The method of producing a non-animal fish and seafood replacement product or food supplement for humans according to claim 4 or claim 5, wherein the cell walls of the micro- or macroalgae are disrupted by physical or enzymatic forces and/or are sterilized prior to their addition to the liquid growth medium.

7. The method of producing a non-animal fish and seafood replacement product or food supplement for humans according to anyone of claims 4 to 6, wherein the water content of the composition is reduced in the range of 0,5 - 95 wt%, preferably 0.5 - 15 wt%, or 3 - 30 wt%, or 0.5 - 15 wt%, or 2 - 9 wt%, or 5 - 25 wt% or 25 - 98 wt%, or 65 - 95 wt% by centrifuging, filtering, pressing and evaporating water by applying heat or reducing atmospheric pressure.

8. The method of producing a non-animal fish and seafood replacement product or food supplement for humans according to anyone of claims 4 to 7, wherein the composition of the fibrous mycelium mass of the edible fungal strain and the edible micro- or macroalgae or parts thereof is fermented in a closed system, preferably a bioreactor or a photobioreactor.

9. The method of producing a non-animal fish and seafood replacement product or food supplement for humans according to anyone of claims 4 to 8, wherein the microalgae are selected from the group consisting of Chlorophyta, Cyanobacteria, Haptophyta, Ochrophyta, Rhodophyta, Trebouxiophyceae, Chlorodentrophyceae, Chlorophyceae, Cyanophyceae, Prymnesiophyceae, Pavlovophyceaea, Bacillariophyceae, Coscinodiscophyceae, Labyrinthulomycetes, Xantophyceae, Eustigmatophyceae, Porphyridiophyceae, Chlorellales, Chlamydomonadales, Sphaeropleales, Chlorodendrales, Oscillatoriales, Synechococcales, Isochrysidales, Bacillariales, Thalassiosirales, Labyrinthulales, Mischocodcales, Eustigmatales, Porphyridiales, Chlorellaceae, Haematococaceae, Dunaliellaceae, Chlamydomonadaceae, Scenedesmaceae, Chlorodendraceae, Chlorodendraceae, Microcoleaceae, Synechococcaceae, Isochrysidaceae, Pavlovaceae, Bacillariaceae, Phaeodactylaceae, Skeletonemataceae, Thalassiosiaceae, Thraustochytriaceae, Pleurochloridaceae, Monodopsidaceae, Porphyridiaceae, Chlorella, Haematococcus, Dunaliella, Scenedesmus, Dimorphus, Acuminatus, Tetraselmis, Arthrospira, Synechococcus, Isochrysidus, Nitzschia, Phaedactylum, Pseudonana, Schizochytrium, Monodus, Nannochloropsis, Porphyridium, preferably from Chlorella vulgaris, Chlorella reinhardtii, Haematococcus pluvialis, Dunaliella salina, Scenedesmus dimorphus and acuminatus, Tetraselmis suecica and striates, Arthrospira platensis, Isochrysidus galbana, Nitzschia dissipata, Phaedactylum tricornutum, Monodus subterranea, Nannochloropsis gaditana, oceanica and oculate, Porphyridium cruentum.

10. The method of producing a non-animal fish and seafood replacement product or food supplement for humans according to anyone of claims 4 to 8, wherein the macroalgae are selected from the group consisting of Rhodophyta, Chlorophyta, Ochrophyta, Bangiophyceae, Florideophyceae, Phaeophyceae, Ulvophyceae, Bangiales, Palmariales, Gigartinales, Ulvales, Laminariales, Bangiaceae, Palmariaceae, Gigartinaceae, Solieriaceae, Ulvaceae, Laminariaceae, Pyropia, Palmaria, Chondrus, Kapaphycus, Eucheuma, Ulva, Sacharina and Laminaria, Bangiaceae yezoensis and haitianensis, Palmaria palmata and mollis, Chondrus crispus, Kapaphycus alvarezii, Euchema cottonii, Ulva lactuca, Sacharina japonica and latissimi, Laminaria digitata.

11. The method of producing a non-animal fish and seafood replacement product or food supplement for humans according to anyone of claims 4 to 10, wherein the at least one edible fungal strain is selected from the group consisting of Basidiomycota, Ascomycota, Pezizomycotina, Agaromycotina, Pezizomycetes, Agaricomycetes, Sordariomycetes, Pezizales, Boletales, Cantharellales, Agaricales, Polyporales, Russulales, Auriculariales, Hypocreales, Morchellaceae, Tuberaceae, Pleurotaceae, Agaricaceae, Marasmiaceae, Cantharellaceae, Hydnaceae, Boletaceae, Meripilaceae, Polyporaceae, Strophariaceae, Lyophyllaceae, Tricholomataceae, Omphalotaceae, Physalacriaceae, Schizophyllaceae, Sclerodermataceae, Ganodermataceae, Sparassidaceae, Hericiaceae, Bondarzewiaceae, Cordycipitaceae, Auriculariaceae, and Fistulinacea, preferably from Pleurotus and most preferably from Pleurotus sapidus.

12. The method of producing a non-animal fish and seafood replacement product or food supplement for humans according to anyone of claims 4 to 11, wherein, after separating the fibrous mycelium mass of the edible fungal strain from the edible micro- or macroalgae or parts thereof the composition is mixed, coated, pasteurized, sterilized, acidified, chilled, frozen, freeze-dried, formed or fermented.

13. A non-animal fish and seafood replacement product or food supplement for humans, obtainable by a method comprising the steps of
a. incubating a composition of mycelia of at least one edible fungal strain and edible micro- or macroalgae or parts thereof in a liquid growth medium,
b. culturing the composition under condition that allow growth of the mycelia to produce a fibrous mycelium mass,
c. separating the fibrous mycelium mass of the edible fungal strain from the edible micro- or macroalgae or parts thereof after completion of the growth of the mycelia to obtain the non-animal fish and seafood replacement product or food supplement for humans.

14. The non-animal fish and seafood replacement product or food supplement according to claim 13, wherein the non-animal fish and seafood replacement product or food supplement is produced according to anyone of claims 4 to 12.

## Patentansprüche

1. Nicht-tierisches Fisch- und Meeresfrüchte-Ersatzprodukt oder Nahrungsergänzungsmittel für Menschen, umfassend eine Zusammensetzung aus
a) einer fibrösen Myzel-Masse wenigstens eines essbaren Pilzstammes im Bereich von 0,5 Gew.-% bis 99,5 Gew.-%
b) Wasser im Bereich von bis zu 99,5 Gew.-%
c) essbaren Mikro- oder Makroalgen oder Teilen davon, wobei die essbaren Mikro- oder Makroalgen oder Teile davon und die fibröse Myzel-Masse zusammen in einem Wachstumsmedium für den essbaren Pilzstamm inkubiert wurden, **dadurch gekennzeichnet, dass** die Zusammensetzung eine oder mehrere der folgenden zusätzlichen Elemente umfasst:
d) ein Geschmackselement, ausgewählt aus Kräutern, Gewürzen oder Extrakten davon, Soßen, natürlichen, künstlichen oder naturidentischen Aromen,
e) ein Texturelement, ausgewählt aus Stärke, Gummi, Elementen auf Algenbasis oder Methyl-Cellulose,
f) ein Konservierungselement, ausgewählt aus Säuren, Salzen, natürlichen Antioxidanten oder künstlichen Konservierungsstoffen,
g) ein Kohlehydratelement auf Basis von Cerealien, Knollen oder Zwiebelknollen,
h) ein Ballaststoffelement, ausgewählt aus Früchten, Algen oder Gemüse,
i) ein Farbelement auf Basis von Gewürzen oder Pflanzenextrakten, Algen, Pflanzenölen, künstliche oder naturidentische Farben oder Pigmenten,
j) ein Proteinelement, ausgewählt aus Soja, Erbse, Weizen, Reis, Lupine, Mungobohne und Kichererbse,
k) ein Lipidelement auf Pflanzenbasis, ausgewählt aus Raps, Leinsamen, Kokosnuss, Canola, Sonnenblumen, Oliven, Algen oder Palm.

2. Nicht-tierisches Fisch- oder Meeresfrüchte-Ersatzprodukt oder Nahrungsergänzungsmittel nach Anspruch 1, wobei die fibröse Myzel-Masse einen Proteingehalt zwischen 15 - 60 Gew.-%, vorzugsweise zwischen 20 - 45 Gew.-% und bevorzugt zwischen 25 - 35 Gew.-%, ein Lipidgehalt zwischen 1 - 15 Gew.-%, vorzugsweise zwischen 2 - 10 Gew.-%, einen Gehalt an einfach und mehrfach ungesättigten Fettsäuren zwischen 0,3 - 10 Gew.-%, vorzugsweise zwischen 1 - 6 Gew.-% und einen Vitamin-D-Gehalt zwischen 0,1 - 500 µg / 100 g, vorzugsweise zwischen 50 - 350 µg / 100 g hat.

3. Nicht-tierisches Fisch- oder Meeresfrüchte-Ersatzprodukt oder Nahrungsergänzungsmittel nach Anspruch 1 oder 2, wobei die essbaren Mikro- oder Makroalgen oder Teile davon und die fibröse Myzel-Masse in der Zusammensetzung zusammen im Wachstumsmedium für den essbaren Pilzstamm für mindestens 60 Minuten inkubiert wurden.

4. Verfahren zur Herstellung eines nicht-tierischen Fisch- und Meeresfrüchte-Ersatzprodukts oder Nahrungsergänzungsmittels für Menschen, umfassend die folgenden Schritte:
a. Inkubation einer Zusammensetzung aus Myzelien wenigstens eines essbaren Pilzstammes und essbaren Mikro- oder Makroalgen oder Teilen davon in einem flüssigen Wachstumsmedium,
b. Kultivierung der Zusammensetzung unter Bedingungen, die das Wachstum der Myzelien zur Bildung einer fibrösen Myzel-Masse ermöglichen,
c. Trennung der fibrösen Myzel-Masse des essbaren Pilzstamms von den essbaren Mikro- oder Makroalgen oder Teilen davon, nachdem die Myzelien vollständig gewachsen sind, um das nicht-tierische Fisch- und Meeresfrüchte-Ersatzprodukt oder Nahrungsergänzungsmittel für Menschen zu erhalten.

5. Verfahren zur Herstellung eines nicht-tierischen Fisch- oder Meeresfrüchte-Ersatzprodukts oder Nahrungsergänzungsmittels für Menschen nach Anspruch 4, wobei zusätzliche Wachstumsfaktoren, Spurenelemente, CO₂, Sauerstoff oder Licht zum flüssigen Wachstumsmedium hinzugefügt wird/werden.

6. Verfahren zur Herstellung eines nicht-tierischen Fisch- oder Meeresfrüchte-Ersatzprodukts oder Nahrungsergänzungsmittels für Menschen nach Anspruch 4 oder 5, wobei die Zellwände der Mikro- oder Makroalgen durch physikalische oder enzymatische Kräfte aufgetrennt und/oder vor dem Hinzufügen zum flüssigen Wachstumsmedium sterilisiert werden.

7. Verfahren zur Herstellung eines nicht-tierischen Fisch- oder Meeresfrüchte-Ersatzprodukts oder Nahrungsergänzungsmittels für Menschen nach einem der Ansprüche 4 bis 6, wobei der Wassergehalt der Zusammensetzung im Bereich von 0,5 - 95 Gew.-%, vorzugsweise 0,5 - 15 Gew.-%, oder 3 - 30 Gew.-%, oder 0,5 - 15 Gew.-%, oder 2 - 9 Gew.-%, oder 5 - 25 Gew.-% oder 25 - 98 Gew.-%, oder 65 - 95 Gew.-% durch Zentrifugieren, Filtern, Pressen oder Verdampfen von Wasser durch die Zufuhr von Hitze oder Reduzierung des atmosphärischen Drucks reduziert wird.

8. Verfahren zur Herstellung eines nicht-tierischen Fisch- oder Meeresfrüchte-Ersatzprodukts oder Nahrungsergänzungsmittels für Menschen nach einem der Ansprüche 4 bis 7, wobei die Zusammensetzung aus der fibrösen Myzel-Masse des essbaren Pilzstammes und den essbaren Mikro- oder Makroalgen oder Teilen davon in einem geschlossenen System, vorzugsweise in einem Bioreaktor oder einem Fotobioreaktor, fermentiert wird.

9. Verfahren zur Herstellung eines nicht-tierischen Fisch- oder Meeresfrüchte-Ersatzprodukts oder Nahrungsergänzungsmittels für Menschen nach einem der Ansprüche 4 bis 8, wobei die Mikroalgen ausgewählt sind aus der Gruppe bestehend aus Chlorophyten, Cyanobakterien, Haptophyta, Ochrophyta, Rhodophyta, Trebouxiophyceae, Chlorodentrophyceae, Chlorophyceae, Cyanophyceae, Prymnesiophyceae, Pavlovophyceaea, Bacillariophyceae, Coscinodiscophyceae, Labyrinthulomycetes, Xantophyceae, Eustigmatophyceae, Porphyridiophyceae, Chlorellales, Chlamydomonadales, Sphaeropleales, Chlorodendrales, Oscillatoriales, Synechococcales, Isochrysidales, Bacillariales, Thalassiosirales, Labyrinthulales, Mischocodcales, Eustigmatales, Porphyridiales, Chlorellaceae, Haematococaceae, Dunaliellaceae, Chlamydomonadaceae, Scenedesmaceae, Chlorodendraceae, Chlorodendraceae, Microcoleaceae, Synechococcaceae, Isochrysidaceae, Pavlovaceae, Bacillariaceae, Phaeodactylaceae, Skeletonemataceae, Thalassiosiaceae, Thraustochytriaceae, Pleurochloridaceae, Monodopsidaceae, Porphyridiaceae, Chlorella, Haematococcus, Dunaliella, Scenedesmus, Dimorphus, Acuminatus, Tetraselmis, Arthrospira, Synechococcus, Isochrysidus, Nitzschia, Phaedactylum, Pseudonana, Schizochytrium, Monodus, Nannochloropsis, Porphyridium, vorzugsweise Chlorella vulgaris, Chlorella reinhardtii, Haematococcus pluvialis, Dunaliella salina, Scenedesmus dimorphus und acuminatus, Tetraselmis suecica und striates, Arthrospira platensis, Isochrysidus galbana, Nitzschia dissipata, Phaedactylum tricornutum, Monodus subterranea, Nannochloropsis gaditana, oceanica and oculate, Porphyridium cruentum.

10. Verfahren zur Herstellung eines nicht-tierischen Fisch- oder Meeresfrüchte-Ersatzprodukts oder Nahrungsergänzungsmittels für Menschen nach einem der Ansprüche 4 bis 8, wobei die Makroalgen ausgewählt sind aus der Gruppe bestehend aus Rhodophyta, Chlorophyta, Ochrophyta, Bangiophyceae, Florideophyceae, Phaeophyceae, Ulvophyceae, Bangiales, Palmariales, Gigartinales, Ulvales, Laminariales, Bangiaceae, Palmariaceae, Gigartinaceae, Solieriaceae, Ulvaceae, Laminariaceae, Pyropia, Palmaria, Chondrus, Kapaphycus, Eucheuma, Ulva, Sacharina und Laminaria, Bangiaceae yezoensis und haitianensis, Palmaria palmata und mollis, Chondrus crispus, Kapaphycus alvarezii, Euchema cottonii, Ulva lactuca, Sacharina japonica und latissimi, Laminaria digitata.

11. Verfahren zur Herstellung eines nicht-tierischen Fisch- oder Meeresfrüchte-Ersatzprodukts oder Nahrungsergänzungsmittels für Menschen nach einem der Ansprüche 4 bis 10, wobei der wenigstens eine essbare Pilzstamm ausgewählt ist aus der Gruppe bestehend aus Basidiomycota, Ascomycota, Pezizomycotina, Agaromycotina, Pezizomycetes, Agaricomycetes, Sordariomycetes, Pezizales, Boletales, Cantharellales, Agaricales, Polyporales, Russulales, Auriculariales, Hypocreales, Morchellaceae, Tuberaceae, Pleurotaceae, Agaricaceae, Marasmiaceae, Cantharellaceae, Hydnaceae, Boletaceae, Meripilaceae, Polyporaceae, Strophariaceae, Lyophyllaceae, Tricholomataceae, Omphalotaceae, Physalacriaceae, Schizophyllaceae, Sclerodermataceae, Ganodermataceae, Sparassidaceae, Hericiaceae, Bondarzewiaceae, Cordycipitaceae, Auriculariaceae, und Fistulinacea, vorzugsweise Pleurotus und bevorzugt Pleurotus sapidus.

12. Verfahren zur Herstellung eines nicht-tierischen Fisch- oder Meeresfrüchte-Ersatzprodukts oder Nahrungsergänzungsmittels für Menschen nach einem der Ansprüche 4 bis 11, wobei nach der Trennung der fibrösen Myzel-Masse des essbaren Pilzstammes von den essbaren Mikro- oder Makroalgen oder Teilen davon die Zusammensetzung gemischt, beschichtet, pasteurisiert, sterilisiert, angesäuert, gekühlt, gefroren, trockengefroren, geformt oder fermentiert wird.

13. Verfahren zur Herstellung eines nicht-tierischen Fisch- oder Meeresfrüchte-Ersatzprodukts oder Nahrungsergänzungsmittels für Menschen, erhältlich durch ein Verfahren bestehend aus den Schritten:
a. Inkubation einer Zusammensetzung aus Myzelien wenigstens eines essbaren Pilzstammes und essbaren Mikro- oder Makroalgen oder Teilen davon in einem flüssigen Wachstumsmedium,
b. Kultivierung der Zusammensetzung unter Bedingungen, die das Wachstum der Myzelien zur Bildung einer fibrösen Myzel-Masse ermöglichen,
c. Trennung der fibrösen Myzel-Masse des essbaren Pilzstammes von den essbaren Mikro- oder Makroalgen oder Teilen davon, nachdem die Myzelien vollständig gewachsen sind, um das nicht-tierische Fisch- und Meeresfrüchte-Ersatzprodukt oder Nahrungsergänzungsmittel für Menschen zu erhalten.

14. Verfahren zur Herstellung eines nicht-tierischen Fisch- oder Meeresfrüchte-Ersatzprodukts oder Nahrungsergänzungsmittels für Menschen nach Anspruch 13, wobei das nicht-tierische Fisch- und Meeresfrüchte-Ersatzprodukt oder Nahrungsergänzungsmittel nach einem der Ansprüche 4 bis 12 hergestellt wird.

## Revendications

1. Produit de substitution ou complément alimentaire à base de poisson non animal et de fruits de mer pour les êtres humains, comprenant une composition constituée par :
a) une masse de mycélium fibreux d'au moins une souche fongique comestible à l'intérieur de la plage de 0,5 % en poids à 99,5 % en poids ;
b) de l'eau à l'intérieur de la plage de jusqu'à 99,5 % en poids ;
c) des micro-algues ou des macro-algues comestibles ou des parties de celles-ci, dans lequel les micro-algues ou les macro-algues comestibles ou les parties de celles-ci et la masse de mycélium fibreux ont été incubées ensemble dans un milieu de croissance pour la souche fongique comestible ;
**caractérisé en ce que** la composition comprend un ou plusieurs des composants additionnels suivants :
d) un composant aromatique sélectionné parmi les fines herbes, les épices ou des extraits de celles-ci, les sauces, les arômes naturels, les arômes artificiels ou les arômes identiques aux arômes naturels ;
e) un composant de texturation sélectionné parmi l'amidon, la gomme, un composant dérivé d'algues ou la cellulose de méthyle ;
f) un composant de conservation sélectionné parmi les acides, les sels, les antioxydants naturels ou les conservateurs artificiels ;
g) un composant de carbohydrate dérivé à partir de céréales, de tubercules ou de bulbo-tubercules ;
h) un composant fibreux sélectionné parmi les fruits, les algues ou les légumes ;
i) un composant de coloration dérivé à partir d'extraits d'épices ou de légumes, d'algues, d'huiles végétales ou de pigments de couleur artificiels ou identiques aux pigments de couleur naturels ;
j) un composant de protéine sélectionné parmi le soja, les pois, le blé, le riz, le lupin, les haricots mungo et les pois chiches ; et
k) un composant de lipide dérivé à partir de plantes sélectionnées parmi le colza, la graine de lin, la noix de coco, le canola, le tournesol, l'olive, les algues et la palme.

2. Produit de substitution ou complément alimentaire à base de poisson non animal et de fruits de mer selon la revendication 1, dans lequel la masse de mycélium fibreux présente une teneur en protéines entre 15 et 60 % en poids, de préférence entre 20 et 45 % en poids et de la façon la plus préférable, entre 25 et 35 % en poids, une teneur en lipides entre 1 et 15 % en poids, de préférence entre 2 et 10 % en poids, une teneur en acides gras mono-insaturés et polyinsaturés entre 0,3 et 10 % en poids, de préférence entre 1 et 6 % en poids, une teneur en vitamine D entre 0,1 et 500 mcg/100 g, de préférence entre 50 et 350 mcg/100 g.

3. Produit de substitution ou complément alimentaire à base de poisson non animal et de fruits de mer selon la revendication 1 ou la revendication 2, dans lequel les micro-algues ou les macro-algues comestibles ou les parties de celles-ci et la masse de mycélium fibreux dans la composition ont été incubées ensemble dans le milieu de croissance pour la souche fongique comestible pendant au moins 60 minutes.

4. Procédé de fabrication d'un produit de substitution ou d'un complément alimentaire à base de poisson non animal et de fruits de mer pour les êtres humains, comprenant les étapes constituées par :
a. l'incubation d'une composition de mycéliums d'au moins une souche fongique comestible et de micro-algues ou de macro-algues comestibles ou de parties de celles-ci dans un milieu de croissance liquide ;
b. la mise en culture de la composition sous une condition qui permet la croissance des mycéliums pour produire une masse de mycélium fibreux ; et
c. la séparation de la masse de mycélium fibreux de la souche fongique comestible vis-à-vis des micro-algues ou des macro-algues comestibles ou des parties de celles-ci après la menée à terme de la croissance des mycéliums pour obtenir le produit de substitution ou le complément alimentaire à base de poisson non animal et de fruits de mer pour les êtres humains.

5. Procédé de fabrication d'un produit de substitution ou d'un complément alimentaire à base de poisson non animal et de fruits de mer pour les êtres humains selon la revendication 4, dans lequel des facteurs de croissance additionnels, des oligoéléments, du CO₂, de l'oxygène ou de la lumière est/sont ajouté(e)(s) au milieu de croissance liquide.

6. Procédé de fabrication d'un produit de substitution ou d'un complément alimentaire à base de poisson non animal et de fruits de mer pour les êtres humains selon la revendication 4 ou la revendication 5, dans lequel les parois des cellules des micro-algues ou des macro-algues sont rompues par des forces physiques ou enzymatiques et/ou sont stérilisées avant leur ajout au milieu de croissance liquide.

7. Procédé de fabrication d'un produit de substitution ou d'un complément alimentaire à base de poisson non animal et de fruits de mer pour les êtres humains selon l'une quelconque des revendications 4 à 6, dans lequel la teneur en eau de la composition est réduite à l'intérieur de la plage de 0,5 à 95 % en poids, de préférence de 0,5 à 15 % en poids ou de 3 à 30 % en poids ou de 0,5 à 15 % en poids ou de 2 à 9 % en poids ou de 5 à 25 % en poids ou de 25 à 98 % en poids ou de 65 à 95 % en poids par centrifugation, par filtrage, par pression et par évaporation de l'eau en appliquant de la chaleur ou une pression atmosphérique réductrice.

8. Procédé de fabrication d'un produit de substitution ou d'un complément alimentaire à base de poisson non animal et de fruits de mer pour les êtres humains selon l'une quelconque des revendications 4 à 7, dans lequel la composition de la masse de mycélium fibreux de la souche fongique comestible et des micro-algues ou des macro-algues comestibles ou des parties de celles-ci est soumise à fermentation dans un système fermé, de préférence un bioréacteur ou un photo-bioréacteur.

9. Procédé de fabrication d'un produit de substitution ou d'un complément alimentaire à base de poisson non animal et de fruits de mer pour les êtres humains selon l'une quelconque des revendications 4 à 8, dans lequel les micro-algues sont sélectionnées parmi le groupe constitué par Chlorophyta, Cyanobacteria, Haptophyta, Ochrophyta, Rhodophyta, Trebouxiophyceae, Chlorodentrophyceae, Chlorophyceae, Cyanophyceae, Prymnesiophyoeae, Pavlovophyceaea, Bacillariophyceae, Coscinodiscophyceae, Labyrinthulomycetes, Xantophyceae, Eustigmatophyceae, Porphyridiophyceae, Chlorellales, Chlamydomonadales, Sphaeropleales, Chlorodendrales, Oscillatoriales, Synechococcales, Isochrysidales, Bacillariales, Thalassiosirales, Labyrinthulales, Mischocodcales, Eustigmatales, Porphyridiales, Chlorellaceae, Haematococaceae, Dunaliellaceae, Chlamydomonadaceae, Scenedesmaceae, Chlorodendraceae, Chlorodendraceae, Microcoleaceae, Synechococcaceae, Isochrysidaceae, Pavlovaceae, Bacillariaceae, Phaeodactylaceae, Skeletonemataceae, Thalassiosiaceae, Thraustochytriaceae, Pleurochloridaceae, Monodopsidaceae, Porphyridiaceae, Chlorella, Haematococcus, Dunaliella, Scenedesmus, Dimorphus, Acuminatus, Tetraselmis, Arthrospira, Synechococcus, Isochrysidus, Nitzschia, Phaedactylum, Pseudonana, Schizochytrium, Monodus, Nannochloropsis, Porphyridium, de préférence parmi Chlorella vulgaris, Chlorella reinhardtii, Haematococcus pluvialis, Dunaliella salina, Scenedesmus dimorphus and acuminatus, Tetraselmis suecica et les striates, Arthrospira platensis, Isochrysidus galbana, Nitzschia dissipata, Phaedactylum tricornutum, Monodus subterranea, Nannochloropsis gaditana, oceanica et oculate, et Porphyridium cruentum.

10. Procédé de fabrication d'un produit de substitution ou d'un complément alimentaire à base de poisson non animal et de fruits de mer pour les êtres humains selon l'une quelconque des revendications 4 à 8, dans lequel les macro-algues sont sélectionnées parmi le groupe constitué par Rhodophyta, Chlorophyta, Ochrophyta, Bangiophyceae, Florideophyceae, Phaeophyceae, Ulvophyceae, Bangiales, Palmariales, Gigartinales, Ulvales, Laminariales, Bangiaceae, Palmariaceae, Gigartinaceae, Solieriaceae, Ulvaceae, Laminariaceae, Pyropia, Palmaria, Chondrus, Kapaphycus, Eucheuma, Ulva, Sacharina et Laminaria, Bangiaceae yezoensis et haitianensis, Palmaria palmata et mollis, Chondrus crispus, Kapaphycus alvarezii, Euchema cottonii, Ulva lactuca, Sacharina japonica et latissimi, et Laminaria digitata.

11. Procédé de fabrication d'un produit de substitution ou d'un complément alimentaire à base de poisson non animal et de fruits de mer pour les êtres humains selon l'une quelconque des revendications 4 à 10, dans lequel l'au moins une souche fongique comestible est sélectionnée parmi le groupe constitué par Basidiomycota, Ascomycota, Pezizomycotina, Agaromycotina, Pezizomycetes, Agaricomycetes, Sordariomycetes, Pezizales, Boletales, Cantharellales, Agaricales, Polyporales, Russulales, Auriculariales, Hypocreales, Morchellaceae, Tuberaceae, Pleurotaceae, Agaricaceae, Marasmiaceae, Cantharellaceae, Hydnaceae, Boletaceae, Meripilaceae, Polyporaceae, Strophariaceae, Lyophyllaceae, Tricholomataceae, Omphalotaceae, Physalacriaceae, Schizophyllaceae, Sclerodermataceae, Ganodermataceae, Sparassidaceae, Hericiaceae, Bondarzewiaceae, Cordycipitaceae, Auriculariaceae, et Fistulinacea, de préférence parmi Pleurotus et de la façon la plus préférable parmi Pleurotus sapidus.

12. Procédé de fabrication d'un produit de substitution ou d'un complément alimentaire à base de poisson non animal et de fruits de mer pour les êtres humains selon l'une quelconque des revendications 4 à 11, dans lequel, après la séparation de la masse de mycélium fibreux de la souche fongique comestible vis-à-vis des micro-algues ou des macro-algues comestibles ou des parties de celles-ci, la composition est mélangée, enrobée, pasteurisée, stérilisée, acidifiée, réfrigérée, congelée, lyophilisée, soumise à formage ou soumise à fermentation.

13. Produit de substitution ou complément alimentaire à base de poisson non animal et de fruits de mer pour les êtres humains, pouvant être obtenu au moyen d'un procédé comprenant les étapes constituées par :
a. l'incubation d'une composition de mycéliums d'au moins une souche fongique comestible et de micro-algues ou de macro-algues comestibles ou de parties de celles-ci dans un milieu de croissance liquide ;
b. la mise en culture de la composition sous une condition qui permet la croissance des mycéliums pour produire une masse de mycélium fibreux ; et
c. la séparation de la masse de mycélium fibreux de la souche fongique comestible vis-à-vis des micro-algues ou des macro-algues comestibles ou des parties de celles-ci après la menée à terme de la croissance des mycéliums pour obtenir le produit de substitution ou le complément alimentaire à base de poisson non animal et de fruits de mer pour les êtres humains.

14. Produit de substitution ou complément alimentaire à base de poisson non animal et de fruits de mer selon la revendication 13, dans lequel le produit de substitution ou le complément alimentaire à base de poisson non animal et de fruits de mer est produit selon l'une quelconque des revendications 4 à 12.
